# EUROPÄISCHE PATENTSCHRIFT

(11) **EP 1 600 129 B1**
(45) Veröffentlichungstag und Bekanntmachung des Hinweises auf die Patenterteilung: **08.08.2007**
(21) Anmeldenummer: 04012434.9
(22) Anmeldetag: 26.05.2004
(51) Int. Cl.: A61F 5/01

(54) **Spreiz-Gehgelenk zur Befestigung zwischen und an den Oberschenkeln eines Patienten**
Abduction joint for walking for attachment between and at the patient's thighs
Articulation d'abduction pour la marche pour la fixation entre et à côté des cuisses

(43) Veröffentlichungstag der Anmeldung: 30.11.2005
(73) Patentinhaber: Horacek, Gregor, 56235 Ransbach-Baumbach (DE)
(72) Erfinder: Horacek, Gregor, 56235 Ransbach-Baumbach (DE)
(74) Vertreter: Quermann, Helmut

(56) Entgegenhaltungen:
- DE-A- 19 934 247
- US-A- 2 690 176
- US-A- 5 362 305

## Beschreibung

Die Erfindung betrifft ein Spreiz-Gehgelenk zur Befestigung zwischen und an den Oberschenkeln eines Patienten, insbesondere eines spastisch gelähmten Kindes oder Jugendlichen.

Bei vielen spastisch gelähmten Kindern und Jugendlichen besteht die Tendenz, dass die Hüftgelenke beim Gehen nach innen rotieren und adduzieren - bis hin zur Überkreuzung der Knie.

Der Tendenz der Innenrotation und Adduktion begegnet man einerseits mit Nachtlagerungsschienen, die die Beine in einer gespreizten Stellung halten. Der Nachteil dieser Schienen besteht darin, dass sie die Beine starr verbinden und somit die Bewegungsfreiheit während des Schlafs einschränken. Eine solche Schiene ist beispielsweise in der EP 0832 623 A1 beschrieben.

Andererseits werden für das Gehen aufwändige, relativ große und kompliziert anzulegende Gehapparate eingesetzt. Bekannt ist eine Spreizvorrichtung gemäß der US 5 147 286 A. Ein wesentlicher Nachteil dieser Spreizvorrichtung besteht darin, dass deren Gelenk, das die Schreitbewegung ermöglichen soll, zu weit vom natürlichen Hüftgelenk positioniert ist, um eine störungsfreie Schreitbewegung zu ermöglichen.

Ähnlich gelagert ist die Problematik bei dem "SU*PER 3 D Hüftgelenk" der Firma Pro-Walk in D-63329 Egelsbach: Das Gelenk dieser Vorrichtung kann bestenfalls in Nähe des Genitalbereichs, aber immer nur unterhalb des Hüftgelenks positioniert werden.

Eine störungsfreie Schreitbewegung ist optimal dann gewährleistet, wenn die Drehpunkte von natürlichem Hüftgelenk und Spreizorthese automatisch und ohne aufwändige Justierung zusammen fallen.

Diese Anforderung erfüllt zum Beispiel die "Hüftabduktionsschiene" der Firma Otto Bock in D-37115 Duderstadt. Zwei Oberschenkelorthesen werden über ein starres Verbindungsstück mit je einem Kugelgelenk an dessen Ende frei beweglich miteinander verbunden. Der Einsatzbereich erstreckt sich jedoch nur über ein Altersspektrum des Patienten von 6-18 Monaten. Andererseits bewirkt die Bewegung des starren Verbindungsstücks zwischen den Orthesenschalen ein Zusammenführen der Knie bei Schreitbewegungen, wodurch diese Hüftabduktionsschiene für das Gehen ungeeignet ist. Desweiteren benötigt die Konstruktion eine relativ große Baubreite und bewirkt so eine starke Abduktion. Dies ist bei entsprechender Indikation und dem hauptsächlich vorgesehenen Einsatzbereich durchaus sinnvoll, ermöglicht aber kein ergonomisches/natürliches Gangbild.

Weiterhin bekannt ist die "Camp SWASH Orthese" der Firma Basko Orthopädie Handelsgesellschaft mbH in D-22761 Hamburg. Hier wird zwar versucht, das Hauptgelenk der Orthese mit dem Hüftgelenk überein zu bringen, dies geschieht aber nicht automatisch, sondern ist abhängig von der konkreten Anbringung/Justierung der notwendigen Hüft-Orthese, auf der diese Gelenke angebracht sind. Außerdem sind die Achsen der Gelenke so ausgerichtet, dass sie bei einer Gehbewegung das Hüftgelenk spreizen, also einer Adduktion entgegen wirken und eine neutrale Gehbewegung nicht zulassen. Ein natürliches Gangbild kann nur dann zustande kommen, wenn Abduktionsmoment der Führungsstäbe und Adduktionsmoment des Spasmus sich gegenseitig aufheben. Dies dürfte nur zufällig vorkommen, zumindest aber äußerst schwierig einstellbar sein.

Der oben genannten Anforderung am nächsten kommt die Spreizvorrichtung gemäß der DE 199 34 247 A1, indem Rotations- und Translationsbewegungen frei kombinierbar sind und somit der theoretische Drehpunkt zwangsläufig und ohne Einjustierung mit dem des natürlichen Hüftgelenks zusammen fällt. Diese Spreizvorrichtung weist aber in der Praxis zwei wesentliche Nachteile auf:
1. Für eine ausreichend große Schreitbewegung müssen die Schienen für die Translationsbewegung sehr lang ausgelegt werden, was die Vorrichtung sperrig macht und bei Endstellung der Schienen auf die Drehverbindung eine hohe Biegebelastung ausübt.
2. Für eine möglichst reibungsarme Translationsbewegung müssen Linear-Kugellager eingesetzt werden. Entsprechend der Vorgabe bezüglich Baubreite und -länge sind diese relativ klein dimensioniert und halten - spätestens beim Einsatz bei Jugendlichen - den hohen "Druck- und Verkantungsbelastungen" in vielen Fällen nicht stand.
In der US-A-2 690 176 ist ein Spreiz-Gehgelenk beschrieben, das in Art einer Kniehebelkonstruktion ausgebildet ist. Diese weist zwei Hebel auf, die um eine freie Achse schwenkbar sind, wobei der jeweilige Hebel im Bereich seines der Achse abgewandten Endes um eine weitere Achse in mit den Oberschenkeln des Patienten verbindbaren Lagerelementen gelagert ist. Ein Spreiz-Gehgelenk mit einer solchen Knickhebelanordnung bewirkt ein völlig anderes Funktions- und Gehverhalten als ein Spreiz-Gehgelenk, das nur einen einzigen Hebel aufweist.

In der US-A-5 362 305 ist ein Spreiz-Gehgelenk zur Befestigung zwischen und an den Oberschenkeln eines Patienten beschrieben, wobei ein einziger Schwenkhebel vorgesehen ist. Dieser ist mittels an seinen Enden befindlichen Drehlagern an mit den Oberschenkeln befestigbaren Orthesen anbringbar ist. Die beiden Orthesen sind auf gleicher Höhe der Oberschenkel des Patienten, somit im gleichen Abstand zum Hüftgelenk des Patienten, angeordnet.

Aufgabe der vorliegenden Erfindung ist es, die Nachteile des vorstehend diskutierten Standes der Technik zu eliminieren und das Spreiz-Gehgelenk zur Befestigung zwischen und an den Oberschenkeln des Patienten zu verbessern.

Gelöst wird die Aufgabe durch ein Spreiz-Gehgelenk, das einerseits gemäß den Merkmalen des Patentanspruches 1, andererseits gemäß den Merkmalen des Patentanspruches 4 ausgebildet ist.

Der Begriff der Orthese ist im Sinne der Erfindung sehr weit gefasst zu verstehen. Bei Orthesen handelt es sich um Hilfsmittel, die die Funktion von Muskeln und Bändern unterstützen, Gelenke führen, korrigierend auf die Beinachsen wirken und ein Längendefizit ausgleichen. Bevorzugt handelt es sich bei der jeweiligen Orthese um eine Orthesenschale, Beinschiene oder Polsterauflage.

Bei dem erfindungsgemäßen Spreiz-Gehgelenk weisen die Drehlager einen Höhenversatz auf, der direkt durch die Länge des Schwenkhebels bestimmt wird. Demgemäß sind die Orthesen an unterschiedlichen Positionen in Relation zum Oberschenkel des Patienten anzubringen. Die eine Orthese ist insbesondere so nah wie möglich am Hüftgelenk, die andere Orthese tiefer, in Richtung Knie, nach Möglichkeit recht nah am Knie anzubringen. Die so angebrachte Konstruktion ermöglicht ein freies Schreiten bei gleichzeitiger Abduktion der Oberschenkel bzw. Knie durch den Schwenkhebel.

Grundsätzlich kann die erfindungsgemäße Spreiz-Gehvorrichtung lediglich aus dem Schwenkhebel bestehen, wenn die direkt am Oberschenkel angebrachten Orthesen die direkte Integration von Lagern entsprechend den Drehlagern zulassen. In der Praxis ist dies allerdings eher aufwendig und unflexibel. Gemäß einer bevorzugten Weiterbildung der Erfindung ist deshalb vorgesehen, dass das Spreiz-Gehgelenk Adapter jedweder Art umfasst, die über die Drehlager mit dem Schwenkhebel bereits verbunden sind. An diesen Adaptern werden die Orthesen befestigt. Dies ist für den Orthopädiemechaniker in jedem Fall mit geringerem Aufwand verbunden und flexibler zu handhaben.

In einer besonderen Weiterbildung der Erfindung sind ein Adapter und das Drehlager nicht unmittelbar mit dem Schwenkhebel verbunden, sondern mittels eines Schlittens. Dieser ist insbesondere am unteren Bereich des Schwenkhebels auf diesem frei verschiebbar. Große Schrittlängen bei relativ kurzem Schwenkhebel oder/und ein relativ weit vom Hüftgelenk platzierter Adapter bewirken einen signifikanten Längenunterschied in der Strecke "oberes Drehlager" - "unteres Drehlager" (als Position am Oberschenkel) im Bewegungsverlauf von Neutralposition zu Schrittposition. Bei einem Schwenkhebel mit fest positionierten Drehlagern ist dieser Längenunterschied nur theoretisch vorhanden, da er durch die Rotation der Hüftgelenksachse um die Körperachse ausgeglichen wird, was allerdings das Gangbild beeinträchtigen kann. Dieser Unterschied kann durch die Bewegung eines Schlittens auf dem Schwenkhebel ausgeglichen werden. Die Bewegung des Schlittens ist vorzugsweise durch einen Anschlag begrenzt, der ein ungewolltes Herausfahren des Schwenkhebels aus dem Schlitten verhindert.

Zur Befestigung und Arretierung an den Orthesen mit entsprechenden Aufnahmen sind die Adapter vorzugsweise trapezförmig gestaltet und am unteren Ende mit einer Arretiereinheit ausgestattet.

Weitere Merkmale der Erfindung sind in den Unteransprüchen, der Beschreibung der Figuren und den Figuren selbst dargestellt, wobei bemerkt wird, dass alle Einzelmerkmale und alle Kombinationen von Einzelmerkmalen weitere erfinderische Ausgestaltungen darstellen.

In den Figuren ist die Erfindung anhand zweier Ausführungsbeispiele erläutert, ohne auf diese beschränkt zu sein.

### Es zeigt:

- Figur 1: eine erste Ausführungsform des Spreiz-Gehgelenks, mit zwei mit dem Schwenkhebel verbundenen Adaptern in einer Stellung, die in etwa der maximalen Spreiz-Geh-Stellung des Patienten entspricht, veranschaulicht in räumlicher Darstellung,
- Figur 2: eine Seitenansicht des in Figur 1 gezeigten Spreiz-Gehgelenks,
- Figur 3: eine Vorderansicht des in den Figuren 1 und 2 gezeigten Spreiz-Gehgelenks,
- Figur 4: die Seitenansicht des in Figur 2 gezeigten Spreiz-Gehgelenks, veranschaulicht ist dessen Neutralposition,
- Figur 5: das in Figur 3 gezeigte Spreiz-Gehgelenk, veranschaulicht in dessen Neutralposition,
- Figur 6: die zweite Ausführungsform des Spreiz-Gehgelenks, unter Verwendung eines Schlittens, in einer Darstellung gemäß Figur 1,
- Figur 7: das in Figur 6 veranschaulichte Spreiz-Gehgelenk in einer Darstellung gemäß Figur 2,
- Figur 8: das in den Figuren 6 und 7 veranschaulichte Spreiz-Gehgelenk in einer Darstellung gemäß Figur 3,
- Figur 9: das in den Figuren 6 bis 8 veranschaulichte Spreiz-Gehgelenk in einer Darstellung gemäß Figur 4,
- Figur 10: das in den Figuren 6 bis 9 gezeigte Spreiz-Gehgelenk in einer Darstellung gemäß Figur 5 und
- Figur 11: eine räumliche Ansicht des in den Figuren 5 bis 10 gezeigten Spreiz-Gehgelenks, bei aus dem Schlitten herausgezogenen Schwenkhebel.

Die Figuren veranschaulichen das erfindungsgemäße Spreiz-Gehgelenk 1 in der Ausführungsform mit den beiden Adaptern 3 und 4. An diesen sind gemäß dieser Ausführungsbeispiele Orthesen anzubringen, die jeweils insbesondere als Orthesenschale, Beinschiene oder Polsterauflage ausgebildet sind.

Das Spreiz-Gehgelenk 1 gemäß der Ausführungsbeispiele weist einen Schwenkhebel 2 auf, an dessen Enden jeweils der Adapter 3 im Drehlager 5 und der Adapter 4 im Drehlager 6 zur Anbindung von Polsterauflagen, Orthesenschalen, Beinschienen oder ähnlich schwenkbar gelagert sind. Die Schwenkachsen der Drehlager 5, 6 sind parallel zueinander angeordnet, insbesondere im wesentlichen parallel zu der durch die Hüftgelenke verlaufenden Achse. Der Schwenkhebel 2 ist somit mit an seinen Enden befindlichen Drehlagern an mit den Oberschenkeln befestigbaren Orthesen bzw. Adaptern 3, 4 angebracht.

In der Neutralposition (entsprechend dem beidbeinigen Stehen mit nebeneinander stehenden Füßen) liegt die Symmetrieachse 7 des Adapters 3, die Symmetrieachse 8 des Adapters 4 und die Symmetrieachse 9 des Schwenkhebels 2 in einer Ebene und die Adapter 3 und 4 weisen einen maximalen Höhenversatz 10 auf, der direkt durch die Länge des Schwenkhebels 2 bestimmt wird. Dem gemäß sind die Adapter 3 und 4 an unterschiedlichen Positionen in Relation zum Oberschenkel des Patienten anzubringen. Der Adapter 3 wäre somit höher, und zwar so nah wie möglich am Hüftgelenk, und der Adapter 4 tiefer, in Richtung Knie anzubringen. Die so angebrachte Konstruktion ermöglicht ein freies Schreiten bei gleichzeitiger Abduktion der Oberschenkel bzw. Knie durch den Schwenkhebel 2. Die Position der Anbindung zu den Drehlagern 5 und 6 ist in Relation zum jeweiligen Adapter unterschiedlich und gewährleistet bei der hier dargestellten Einsatzweise, dass bei gegebener Länge des Schwenkhebels 2 einerseits die Gesamtlänge der Konstruktion möglichst minimal bleibt und andererseits das über den Schwenkhebel 2 erzeugte Moment an den Außenflächen der plattenförmigen Adapter 3 und 4 mit möglichst geringer Kraft aufgenommen werden kann. In diesem Sinne ist vorgesehen, dass beim näher zum Hüftgelenk positionierten Adapter 3 dessen Anbindung 11 zum Drehlager 5 in dessen oberen Bereich, und beim näher zum Knie positionierten Adapter 4 dessen Anbindung 12 zum Drehlager 6 in dessen unteren Bereich angebracht ist.

Der Schwenkhebel 2 pendelt während der Schreitbewegung zusammen mit dem Adapter 4 in Drehlager 5 um die Symmetrieachse 7 des Adapters 3. Die freie Verschwenkbarkeit des Adapters 4 im Drehlager 6 richtet den Schnittpunkt der Symmetrieachsen 7 und 8 automatisch auf das Hüftgelenk aus und ermöglicht so eine störungsfreie Schreitbewegung. Entsprechend den hohen Adduktions- und Rotationskräften - die Innenrotation der Hüftgelenke führt die Knie zusammen - sind der Schwenkhebel 2 und die Drehlager 5 und 6 gegen Biegebelastung und Torsion stabil auszulegen.

Das Maß der Abduktion wird in der hier dargestellten Ausführung durch die Verkröpfung des Schwenkhebels 2 und die Dicke der Distanzscheiben 13 zwischen dem Schwenkhebel 2 und den Adaptern 3 und 4 bestimmt.

Grundsätzlich kann die erfindungsgemäße Spreiz-Gehvorrichtung 1 lediglich aus dem Schwenkhebel 2 bestehen, wenn die direkt am Oberschenkel angebrachten Orthesen - Orthesenschalen, Beinschienen, Polsterauflagen oder ähnlich - die direkte Integration von Lagern entsprechend den Drehlagern 5 und 6 zulassen. Die Adapter 3 und 4 würden in diesem Fall direkt durch die Orthesen ersetzt. Ein Spreiz-Gehgelenk mit Adaptern 3 und 4 jedweder Art, die über Drehlager 5 und 6 mit dem Schwenkhebel 2 bereits verbunden sind, ist für den Orthopädiemechaniker in jedem Fall mit geringerem Aufwand und flexibler zu handhaben.

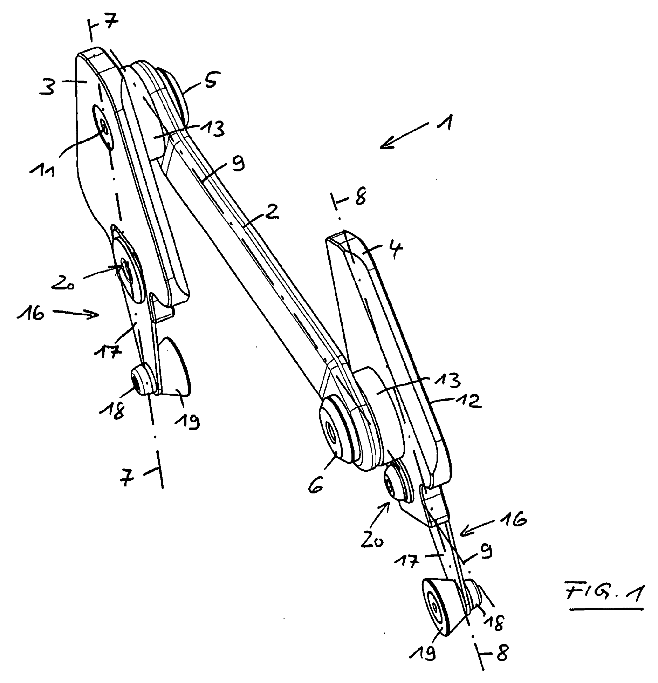

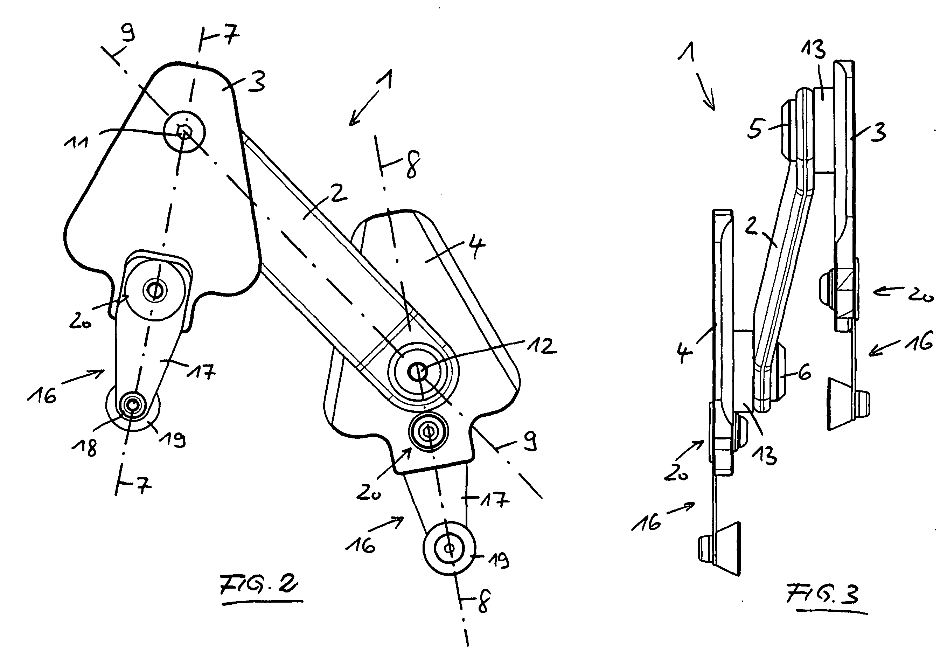

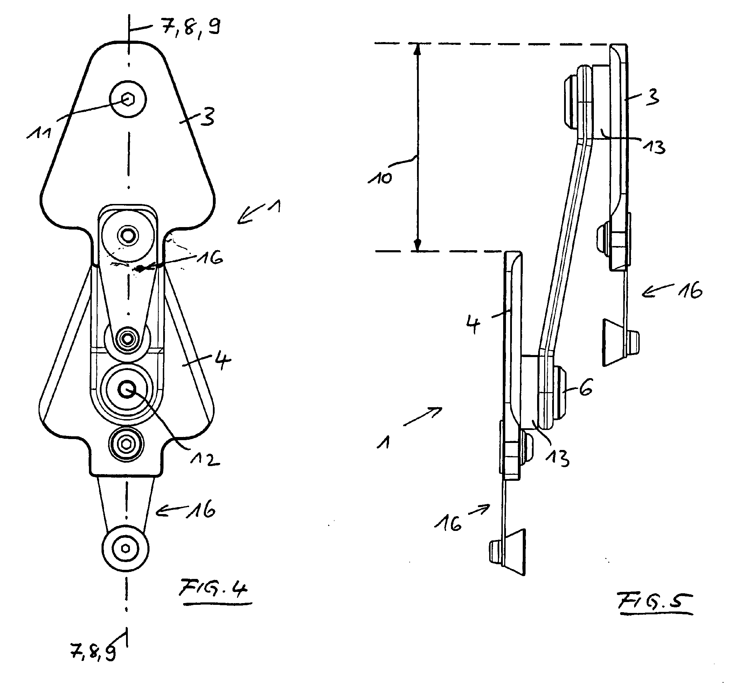

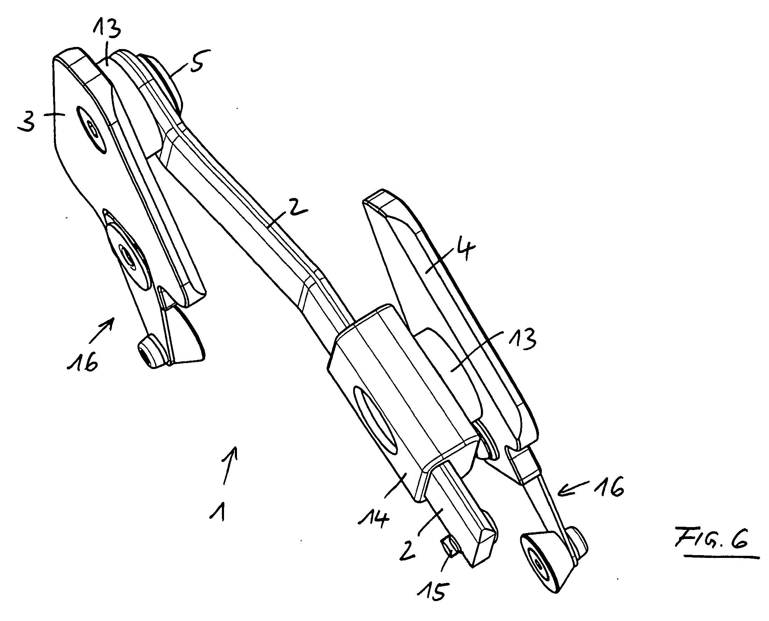

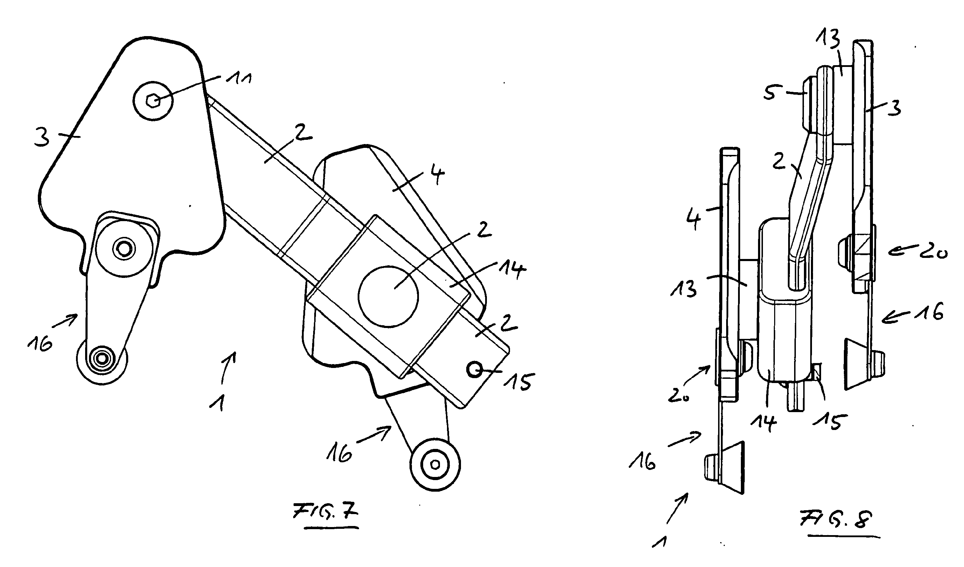

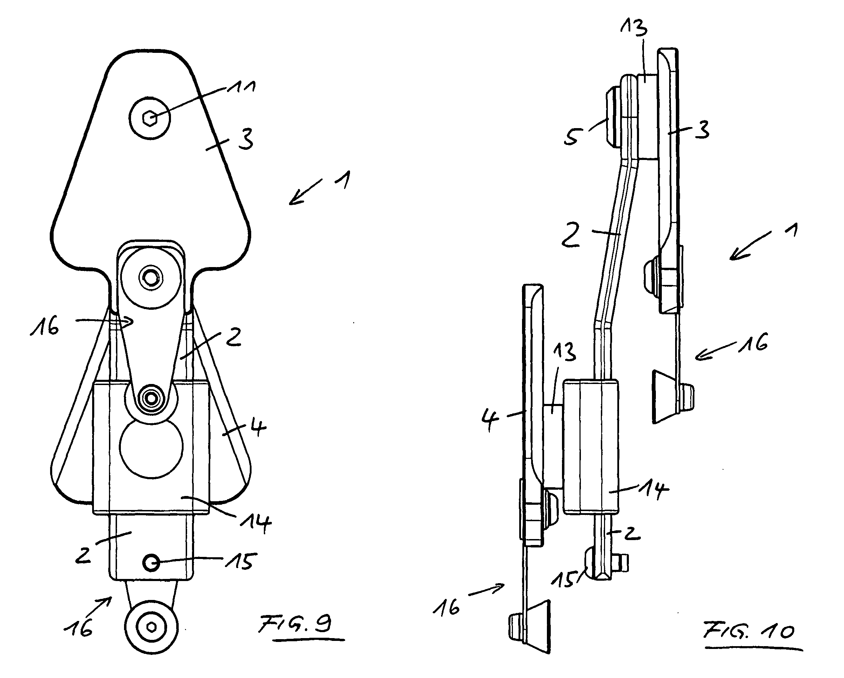

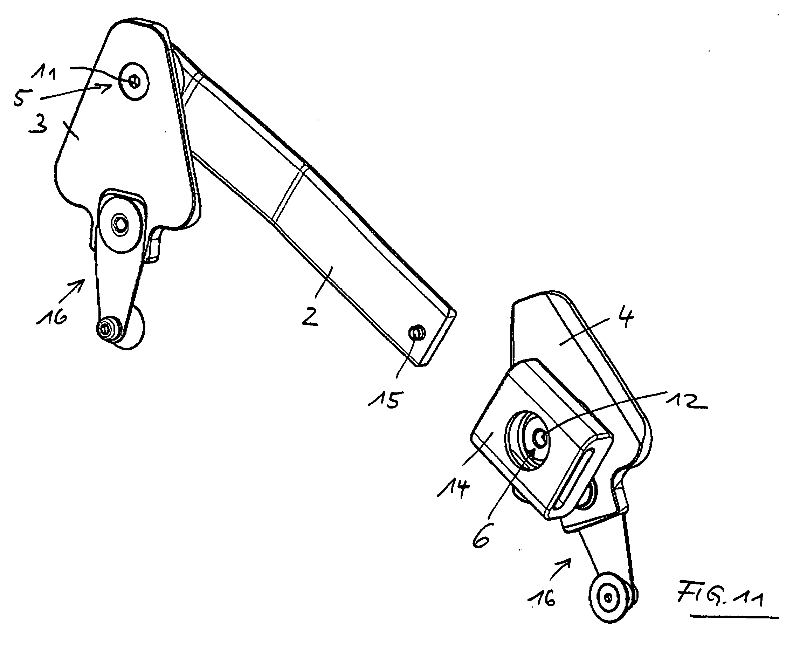

Bei der besonderen Ausführungsform der Erfindung gemäß der Darstellung in den Figuren 6 bis 11 sind der Adapter 4 und das Drehlager 6 nicht unmittelbar mit dem Schwenkhebel 2 verbunden, wie es zur Ausführungsform nach den Figuren 1 bis 5 dargestellt ist, sondern mittels eines Schlittens 14, der im unteren Bereich des Schwenkhebels 2 auf diesem frei verschiebbar ist. Große Schrittlängen bei relativ kurzem Schwenkhebel 2 oder/und ein relativ weit vom Hüftgelenk platzierter Adapter 3 bewirken einen signifikanten Längenunterschied in der Strecke "oberes Drehlager 5" - "unteres Drehlager 6" (als Position am Oberschenkel) im Bewegungsverlauf von Neutralposition zu Schrittposition. Bei einem Schwenkhebel 2 mit fest positionierten Drehlagern 5, 6 ist dieser Längenunterschied nur theoretisch vorhanden, da er durch die Rotation der Hüftgelenksachse um die Körperachse ausgeglichen wird, was allerdings das Gangbild beeinträchtigen kann. Dieser Unterschied kann durch die Bewegung des Schlittens 14 auf dem Schwenkhebel 2 ausgeglichen werden. Die Bewegung des Schlittens 14 ist nach unten durch einen Anschlag 15 begrenzt und verhindert so ein ungewolltes "Herausfahren" des Schwenkhebels 2 aus dem Schlitten 14.

Zur Befestigung und Arretierung an entsprechenden Aufnahmen der Orthesen sind die Adapter 3 und 4 trapezförmig gestaltet und am unteren Ende mit einer Arretiereinheit 16 ausgestattet, die im wesentlichen aus einem Federblatt 17, einem Arretierbolzen 18 und einem Auslöser 19 besteht. Die jeweilige Arretiereinheit ist mittels einer Schraubverbindung 20 mit dem zugeordneten Adapter 3 bzw. 4 verbunden.

## Patentansprüche

1. Spreiz-Gehgelenk (1) zur Befestigung zwischen und an den Oberschenkeln eines Patienten, wobei ein Schwenkhebel (2) mit an seinen Enden befindlichen Drehlagern (5, 6) an mit den Oberschenkeln befestigbaren Orthesen angbringbar ist, wobei ein einziger Schwenkhebel (2) vorgesehen ist, **dadurch gekennzeichnet, dass** im Gebrauch des Spreiz-Gehgelenks (1) eines der Drehlager (5) näher zum Hüftgelenk des Patienten und das andere Drehlager (6) näher zum Kniegelenk des Patienten positioniert ist, wobei die Schwenkachsen der Drehlager (5, 6) im Wesentlichen parallel zu der durch die Hüftgelenke verlaufenden Achse angeordnet sind.

2. Gelenk nach Anspruch 1, **dadurch gekennzeichnet, dass** den Drehlagern (5, 6) des Schwenkhebels (2) Adapter (3, 4) zugeordnet sind, die der Anbindung der Orthesen dienen.

3. Gelenk nach einem der Ansprüche 1 oder 2, **dadurch gekennzeichnet, dass** die Adapter (3, 4)/ die Orthesen direkt in den Drehlagern (5, 6) des Schwenkhebels (2) gelagert sind.

4. Spreiz-Gehgelenk (1) zur Befestigung zwischen und an den Oberschenkeln eines Patienten, wobei ein Schwenkhebel (2) schwenkbar an mit den Oberschenkeln befestigbaren Orthesen anbringbar ist, wobei die Schwenkbarkeit des Schwenkhebels (2) mittels zweier Drehlager (5, 6) bewerkstelligt wird und wobei ein einziger Schwenkhebel (2) vorgesehen ist, **dadurch gekennzeichnet, dass** im Gebrauch des Spreiz-Gehgelenks (1) eines der Drehlager (5) näher zum Hüftgelenk des Patienten und das andere Drehlager (6) näher zum Kniegelenk des Patienten positioniert ist, wobei die Schwenkachsen der Drehlager (5, 6) im Wesentlichen parallel zu der durch die Hüfgelenke verlaufenden Achse angeordnet sind, wobei ferner eine der Orthesen oder ein Adapter (3), der der Anbindung dieser Orthese dient, direkt in einem Drehlager (5) des Schwenkhebels (2) gelagert ist und die andere Orthese oder ein Adapter (4), der der Anbindung der anderen Orthese dient, in einem Drehlager (6) eines Schlittens (14) gelagert ist, wobei der Schlitten (14) in einem Endbereich des Schwenkhebels (2) auf diesem frei verschiebbar ist.

5. Gelenk nach Anspruch 4, **dadurch gekennzeichnet, dass** der Schlitten (14) in dem dem Kniegelenk zugewandten Bereich des Schwenkhebels (2) angeordnet ist.

6. Gelenk nach Anspruch 4 oder 5, **dadurch gekennzeichnet, dass** die Bewegung des Schlittens (14) in Richtung des diesem zugewandten Schwenkhebelendes durch einen Anschlag (15) begrenzt ist.

7. Gelenk nach einem der Ansprüche 1 bis 6, **dadurch gekennzeichnet, dass** die jeweilige Orthese als Orthesenschale, Beinschiene oder Polsterauflage ausgebildet ist.

8. Gelenk nach einem der Ansprüche 1 bis 7, **dadurch gekennzeichnet, dass** der jeweilige Adapter (3, 4) trapezförmig gestaltet ist.

9. Gelenk nach einem der Ansprüche 1 bis 8, **dadurch gekennzeichnet, dass** der jeweilige Adapter (3, 4) eine Arretiereinheit (16) für die Orthese aufweist.

10. Gelenk nach einem der Ansprüche 1 bis 9, **dadurch gekennzeichnet, dass** Symmetrieachsen (7, 8, 9) der beiden Adapter (3, 4) und des Schwenkhebels (2) in der Neutralposition des Patienten, bei beidbeinigem Stehen mit nebeneinander stehenden Füßen, in einer Ebene angeordnet sind.

11. Gelenk nach einem der Ansprüche 1 bis 10, **dadurch gekennzeichnet, dass** die Anbindung des näher zum Hüftgelenk positionierten Adapters (3) in dessen dem Hüftgelenk zugewandten Bereich und die Anbindung des näher zum Kniegelenk positionierten Adapters (4) in dessen dem Kniegelenk zugeordneten Bereich erfolgt.

12. Gelenk nach einem der Ansprüche 1 bis 11, **dadurch gekennzeichnet, dass** der Schwenkhebel (2) im Bereich seiner Enden gekröpft ausgebildet ist.

## Claims

1. Abduction joint (1) for walking, designed to be secured between and at a patient's thighs, in which a pivot lever (2) with rotary bearings (5, 6) located at its ends can be fitted to orthoses that can be secured to the thighs, a single pivot lever (2) being provided, **characterized in that**, during use of the abduction joint (1) for walking, one of the rotary bearings (5) is positioned nearer to the hip joint of the patient, and the other rotary bearing (6) is positioned nearer to the knee joint of the patient, the pivot axes of the rotary bearings (5, 6) being arranged substantially parallel to the axis extending through the hip joints.

2. Joint according to Claim 1, **characterized in that** the rotary bearings (5, 6) of the pivot lever (2) are assigned adapters (3, 4), which serve for attachment of the orthoses.

3. Joint according to either of Claims 1 and 2, **characterized in that** the adapters (3, 4)/the orthoses are mounted directly in the rotary bearings (5, 6) of the pivot lever (2).

4. Abduction joint (1) for walking, designed to be secured between and at a patient's thighs, in which a pivot lever (2) can be fitted pivotably on orthoses that can be secured to the thighs, the pivotability of the pivot lever (2) being obtained by means of two rotary bearings (5, 6), with a single pivot lever (2) being provided, **characterized in that**, during use of the abduction joint (1) for walking, one of the rotary bearings (5) is positioned nearer to the hip joint of the patient, and the other rotary bearing (6) is positioned nearer to the knee joint of the patient, the pivot axes of the rotary bearings (5, 6) being arranged substantially parallel to the axis extending through the hip joints, and in which, moreover, one of the orthoses or an adapter (3), which serves for attachment of this orthosis, is mounted directly in a rotary bearing (5) of the pivot lever (2), and the other orthosis or an adapter (4), which serves for attachment of the other orthosis, is mounted in a rotary bearing (6) of a slide (14), the slide (14) being freely displaceable on the pivot lever (2) in an end area thereof.

5. Joint according to Claim 4, **characterized in that** the slide (14) is arranged in the area of the pivot lever (2) directed towards the knee joint.

6. Joint according to Claim 4 or 5, **characterized in that** the movement of the slide (14), in the direction of the pivot lever end facing towards it, is limited by a limit stop (15).

7. Joint according to one of Claims 1 to 6, **characterized in that** the respective orthosis is designed as an orthosis shell, a leg splint or a padded support.

8. Joint according to one of Claims 1 to 7, **characterized in that** the respective adapter (3, 4) has a trapezoid shape.

9. Joint according to one of Claims 1 to 8, **characterized in that** the respective adapter (3, 4) has a locking unit (16) for the orthosis.

10. Joint according to one of Claims 1 to 9, **characterized in that** axes of symmetry (7, 8, 9) of the two adapters (3, 4) and of the pivot lever (2) are arranged in one plane in the neutral position of the patient, with the latter standing on both legs, and with both feet placed next to one another.

11. Joint according to one of Claims 1 to 10, **characterized in that** the adapter (3) positioned nearer to the hip joint is attached in its area directed towards the hip joint, and the adapter (4) positioned nearer to the knee joint is attached in its area directed towards the knee joint.

12. Joint according to one of Claims 1 to 11, **characterized in that** the pivot lever (2) is bent at an angle in the area of its ends.

## Revendications

1. Articulation d'abduction pour la marche (1) pour la fixation entre les cuisses et à côté des cuisses d'un patient, un levier pivotant (2) avec un coussinet de pivotement (5, 6) au niveau de l'une de ses extrémités pouvant être installé aux orthèses fixables avec les cuisses, un levier pivotant (2) unique étant prévu, **caractérisée en ce que**, lors de l'utilisation de l'articulation d'abduction pour la marche (1), l'un des coussinets de pivotement (5) est positionné plus près de l'articulation de la hanche du patient et l'autre coussinet de pivotement (6) est positionné plus près de l'articulation du genou du patient, les axes de pivotement des coussinets de pivotement (5, 6) étant ordonnés de façon essentiellement parallèle à l'axe passant par l'articulation de la hanche.

2. Articulation selon la revendication 1, **caractérisée en ce que** les coussinets de pivotement (5, 6) du levier de pivotement (2) sont reliés à des adaptateurs (3, 4) servant à relier les orthèses.

3. Articulation selon l'une des revendications 1 ou 2, **caractérisée en ce que** les adaptateurs (3, 4)/ les orthèses sont monté(e)s directement dans les coussinets de pivotement (5, 6) du levier de pivotement (2).

4. Articulation d'abduction pour la marche (1) pour la fixation entre les cuisses et à côté des cuisses d'un patient, un levier pivotant (2) pouvant être installé pivotant au niveau d'orthèses fixables avec les cuisses, la possibilité de pivotement du levier de pivotement (2) étant réalisée au moyen de deux coussinets de pivotement (5, 6) et un levier pivotant (2) unique étant prévu, **caractérisée en ce que**, lors de l'utilisation de articulation d'abduction pour la marche (1), l'un des coussinets de pivotement (5) est positionné plus près de l'articulation de la hanche du patient et l'autre coussinet de pivotement (6) est positionné plus près de l'articulation du genou du patient, les axes de pivotement des coussinets de pivotement (5, 6) étant ordonnés de façon essentiellement parallèle à l'axe passant par l'articulation de la hanche, et l'une des orthèses ou un adaptateur (3), servant à relier cette orthèse, étant en outre monté(e) directement dans un coussinet de pivotement (5) du levier de pivotement (2) et l'autre orthèse ou un adaptateur (4), servant à relier l'autre orthèse, étant monté(e) dans un coussinet de pivotement (6) d'un coulisseau (14), le coulisseau (14) pouvant être librement décalé vers une zone terminale du levier pivotant (2).

5. Articulation selon la revendication 4, **caractérisée en ce que** le coulisseau (14) est ordonné dans la zone du levier de pivotement (2) orientée vers l'articulation du genou.

6. Articulation selon la revendication 4 ou 5, **caractérisée en ce que** le mouvement du coulisseau (14) est limité par une butée (15) en direction de l'extrémité du levier de pivotement orientée vers le coulisseau.

7. Articulation selon l'une des revendications 1 à 6, **caractérisée en ce que** les orthèses respectives sont configurées sous forme de coque d'orthèse, d' attelle ou de rembourrage.

8. Articulation selon l'une des revendications 1 à 7, **caractérisée en ce que** les adaptateurs respectifs (3, 4) sont configurés de façon trapézoïdale.

9. Articulation selon l'une des revendications 1 à 8, **caractérisée en ce que** l'adaptateur respectif (3, 4) comporte une unité d'arrêt (16) pour l'orthèse.

10. Articulation selon l'une des revendications 1 à 9, **caractérisée en ce que** les axes de symétrie (7, 8, 9) des deux adaptateurs (3, 4) et du levier pivotant (2), lorsque le patient est en position neutre, campé sur des deux jambes avec les pieds situés à côté l'un de l'autre, sont ordonnés sur un niveau.

11. Articulation selon l'une des revendications 1 à 10, **caractérisée en ce que** la liaison de l'adaptateur (3) positionné plus près de l'articulation de la hanche est réalisée dans sa zone orientée vers l'articulation de la hanche et la liaison de l'adaptateur (4) positionné plus près de l'articulation du genou dans sa zone orientée vers l'articulation du genou.

12. Articulation selon l'une des revendications 1 à 11, **caractérisée en ce que** le levier pivotant (2) est coudé dans la zone de ses extrémités.
